# FASCICULE DE BREVET EUROPEEN

(11) **EP 0 623 329 B1**
(45) Date de publication et mention de la délivrance du brevet: **07.07.1999**
(21) Numéro de dépôt: 94400966.1
(22) Date de dépôt: 04.05.1994
(51) Int. Cl.: A61F 9/00

(54) **Dispositif de pose d'une sonde ophtalmologique**
Durchfädelungsvorrichtung einer ophthalmologischen Sonde
Ophthalmological intubation device

(30) Priorité: 04.05.1993 FR 9305277
(43) Date de publication de la demande: 09.11.1994
(73) Titulaire: F.C.I.(FRANCE CHIRURGIE INSTRUMENTATION) S.A., F-92130 Issy-Les Moulineaux (FR)
(72) Inventeur: Ritleng, Pierre, F-06000 Nice (FR)
(74) Mandataire: Faber, Jean-Paul

(56) Documents cités:
- EP-A- 0 238 018
- WO-A-83/03766
- AT-B- 316 011
- US-A- 3 948 272
- US-A- 4 380 239

## Description

La présente invention a pour objet un dispositif de pose d'une sonde ophtalmologique bicanaliculaire.

Les sondes bicanaliculaires sont constituées par un tube fin de silicone destiné à être intubé dans les canalicules lacrymaux en vue de remédier à certains dysfonctionnements tels des sténoses qui obturent lesdits canalicules. Les canalicules correspondent avec l'oeil par des méats disposés au voisinage du nez dans les paupières supérieure et inférieure et ils ont pour fonction d'évacuer le liquide lacrymal par le nez.

Il est connu, en cas de pathologie des canalicules, d'intuber ceux-ci par un fil ou tube souple qui peut débloquer les sténoses et rétablir la circulation lacrymale. Le problème qui est résolu par l'invention est celui de l'intubation des voies lacrymales . Actuellement, le tube de silicone à introduire dans les canalicules est terminé, à chacune de ses extrémités, par un mandrin métallique souple qui sert de guide pour parcourir un parcours tourmenté. L'inconvénient de cette pose est qu'elle est le plus souvent sanglante, pour sortir les mandrins de la cavité nasale et, en tout état de cause traumatisante. Un tel procédé est décrit dans US-A- 4 380 239.

Pour remédier à ce problème, il est possible d'employer la technique de BUSSE, avec une sonde de JÜNEMANN. La technique de BUSSE consiste essentiellement, à faire passer dans les canalicules un fil fin, puis ensuite à tirer à l'aide de ce fil fin le tube de silicone qui est de diamètre plus important.

La sonde de JÜNEMANN a la forme d'une canule creuse présentant, à sa partie supérieure des ailettes de préhension et, à sa partie inférieure, un trou latéral, l'extrémité avant étant fermée. L'extrémité avant de la sonde est, dans un premier temps, introduite dans le méat en position légèrement inclinée sur l'horizontale pour pénétrer dans la partie horizontale du canalicule, puis on lui fait subir une rotation de sensiblement 90° et l'on pousse un fil fin de guidage, par exemple en prolène, à l'intérieur de la sonde, puis de la cavité nasale, dans laquelle il peut être repris à l'aide d'une pince ou d'un crochet . La sonde de JÜNEMANN est alors glissée de l'autre coté du fil d'où elle s'échappe. Cette opération est répétée dans chacun des deux canalicules. Après quoi les fils de guidage sont chacun réunis aux extrémités de la sonde proprement dite.

Cette technique présente l'avantage d'être exsangue et non traumatisante. Par contre, par rapport aux sondes bicanaliculaires mentionnées ci-dessus, elle exige davantage de temps et est nettement plus complexe. le temps nécessaire est en particulier dû au fait que le chirurgien doit effectuer la liaison fil de prolène- tube de silicone après avoir extrait la sonde.
Le chirurgien doit donc effectuer successivement les opérations suivantes:
- a) introduire la canule dans une voie lacrymale;
- b) introduire, par poussée, le fil de guidage dans la canule;
- c) tirer le fil guide à l'intérieur de la cavité nasale;
- d) extraire la canule de la voie lacrymale et du fil en la faisant glisser sur le fil guide, vers le haut;
- e) réunir le fil de guidage et le tube de silicone;
- f) faire progresser l'ensemble fil de guidage-tube de silicone dans la voie lacrymale en tirant vers le bas et en poussant de haut en bas par des petits mouvements successifs pour éviter une désolidarisation du fil et du tube;
- g) Lorsque le tube de silicone apparaît dans la cavité nasale, séparer le fil du tube et tirer sur celui-ci.

Après quoi, la même opération doit être répétée dans l'autre méat.

L'opération la plus longue, et la plus délicate est l'étape f). Il n'est pas possible pour le chirurgien de fixer le fil de guidage sur le tube silicone, étant donné que la canule doit être dégagée du fil de guidage avant que cette liaison soit réalisée.

La présente invention a pour objet de pallier cet inconvénient et de permettre la mise à la disposition du chirurgien d'un ensemble fil de guidage-tube de silicone duquel la sonde puisse être extraite aisément en évitant ou facilitant les opérations e, f et g.

Selon la présente invention, le dispositif de pose d'une sonde ophtalmologique bicanaliculaire, comprenant un tube de silicone constituant la sonde proprement dite, et une canule d'intubation présentant un corps cylindrique creux, fermé à sa partie avant mais avec une lumière latérale, dont le diamètre extérieur est inférieur au diamètre extérieur du tube de silicone, le corps cylindrique présentant une fente longitudinale s'étendant de la lumière jusqu'à l'extrémité postérieure du corps, est caractérisé en ce que: La fente longitudinale est de largeur inférieure à la largeur de la lumière, le tube de silicone étant muni, à chacune de ses extrémités, d'un fil de guidage, de diamètre inférieur au diamètre interne du corps cylindrique creux et à la largeur de la lumière, dans le fil de guidage étant formée, au voisinage de sa liaison avec le tube, une zone rétrécie dont le diamètre est légèrement inférieur à la largeur de la fente sur une longueur sensiblement égale à la longueur de la fente de la canule.

La pose d'une sonde bicanaliculaire constituée par un tube de silicone, à l'aide d'une canule d'intubation selon l'invention consiste à:
- insérer la canule dans un méat;
- faire glisser, à partir de la canule, le fil de guidage dans le canalicule jusqu'à ce qu'il devienne apparent dans le nez;
- faire passer la canule sur la partie rétrécie pour la dégager du fil de guidage.
Il suffit donc de faire glisser la canule sur le fil jusqu'à ce que la zone de moindre épaisseur arrive en face de la fente et de dégager la canule en la faisant pivoter par rapport au fil.

Grâce à l'invention, la liaison entre le fil de guidage en prolène et le tube de silicone est effectuée en usine, par exemple par collage ( étape e) et le chirurgien peut tirer sur le fil de guidage apparaissant dans le nez , d'un mouvement quasiment continu ce qui permet de gagner un temps important (étape f).

D'autres caractéristiques et avantages de l'invention apparaîtront au cours de la description qui va suivre d'un mode particulier de réalisation, donné uniquement à titre d'exemple non limitatif en regard des dessins qui représentent :
- La Figure 1 une vue par-dessus d'une sonde selon l'invention;
- La Figure 2, une vue de la liaison tube de silicone-fil de prolène;
- La Figure 3, une vue d'une sonde à l'intérieur d'un canalicule.

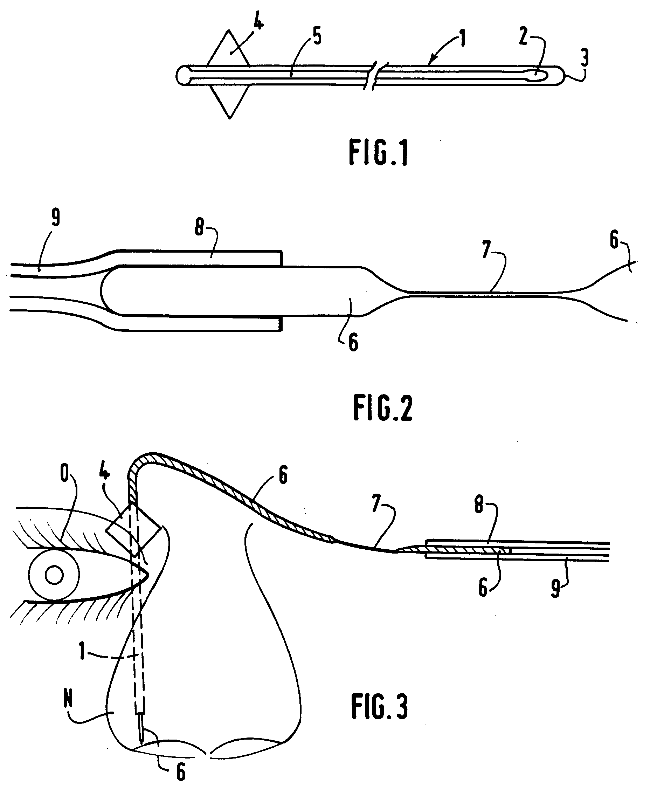

Sur la figure 1, on voit que la canule se compose d'un corps creux cylindrique 1 dont l'extrémité inférieure ou avant 3 est fermée. Au voisinage de cette extrémité est percée une lumière ou ouverture oblongue 2. De l'autre coté de la canule se trouvent des ailettes 4 qui constituent des organes de préhension.

Conformément à l'invention, une fente longitudinale 5 s'étend à partir de la lumière 2 sur toute la longueur de la canule. Cette fente est réalisée par exemple par électroérosion.

Compte tenu des contraintes de type anatomiques, la canule a un diamètre de 0,5 mm, et la fente une largeur de 0,25 mm. La longueur de la canule est de 100 mm, cette dimension n'étant pas critique.

Dans cette canule, on insère un fil de prolène 6 ( FIG.2) dont le diamètre est de 0,4 mm de manière à ce qu'il puisse aisément coulisser à l'intérieur de la canule. Comme cela apparaît sur la figure 2, le fil de prolène est relié à un tube de silicone 9 dans la partie avant 8 duquel il est inséré à force et éventuellement collé. Le diamètre interne du tube 9 est de 0,3 mm et, après insertion du fil 6 le diamètre extérieur est de 0,64 mm ,par exemple.

On retrouve les éléments mentionnés ci-dessus sur la figure 3. La canule 1 est introduite dans le canalicule inférieur de l'oeil O et elle pénètre dans la cavité nasale N. Lors de cette insertion, la canule 1 contient du fil 6 jusqu'au niveau de la lumière 2. Après cette insertion, on pousse le fil 6 à l'aide d'une pince (non représentée) jusqu'à ce que son extrémité inférieure apparaisse à l'intérieur du nez. Tout en maintenant ce fil, on dégage la canule 1 du canalicule et on la fait glisser sur le fil 6 jusqu'au niveau de la zone rétrécie 7 de celui-ci. Par un mouvement de pivotement, elle alors dégagée du fil 6 et celui-ci est tiré vers le bas en entraînant le tube 9 qui peu à peu pénètre à son tour dans le canalicule concerné.
On répète la même opération avec le second canalicule et l'opération est terminée en effectuant une ligature des deux extrémités du tube 9 à l'intérieur du nez.

## Revendications

1. Dispositif de pose d'une sonde ophtalmologique bicanaliculaire, comprenant un tube de silicone (9) constituant la sonde proprement dite, et une canule d'intubation présentant un corps cylindrique creux (1), fermé à sa partie avant (3) mais avec une lumière latérale (2), dont le diamètre extérieur est inférieur au diamètre extérieur (9) du tube de silicone, le corps cylindrique (1) présentant une fente longitudinale (5) s'étendant de la lumière (2) jusqu'à l'extrémité postérieure du corps (1),
caractérisé en ce que:
le tube de silicone (9) est muni à chacune de ses extrémités d'un fil de guidage (6), de diamètre inférieur au diamètre interne du corps cylindrique creux (1) et à la largeur de la lumière (2), une zone rétrécie (7), dont le diamètre est légèrement inférieur à la largeur de la fente (5) étant formée sur une longueur sensiblement égale à la longueur de la fente (5) de la canule, dans le fil de guidage (6), au voisinage de sa liaison (8) avec le tube (9).

## Claims

1. A device for putting a bicanalicular ophthalmological probe in place, comprising a silicone tube (9) constituting the probe proper, and an intubation cannula exhibiting a hollow cylindrical body (1) closed in the front part (3) of it but having in the side an opening (2) of a smaller outer diameter than the outer diameter (9) of the silicone tube, the cylindrical body (1) exhibiting a longitudinal slit (5) extending from the opening (2) back to the rear end of the body (1), characterized in that the silicone tube (9) is equipped at each end with a guide-wire (6) of a diameter less than the inner diameter of the hollow cylindrical body (1) and than the width of the opening (2), whilst a zone (7) constricted to a diameter slightly less than the width of the slit (5) is formed in the guide-wire (6) in the vicinity of its junction (8) with the tube (9), over a length substantially equal to the length of the slit (5).

## Patentansprüche

1. Vorrichtung zum Setzen einer ophtalmologischen Bikanalsonde mit einem die eigentliche Sonde bildenden Siliconröhrchen (9) und einer Intubationskanüle in Form eines zylindrischen Hohlkörpers (1), der an seinem vorderen Abschnitt (3) verschlossen, aber mit einem seitlichen Loch (2) versehen ist und dessen Außendurchmesser kleiner als der Außendurchmesser (9) des Siliconröhrchens ist, wobei der zylindrische Körper (1) einen längsverlaufenden Schlitz (5) aufweist, der sich von dem Loch (2) bis zu dem hinteren Ende des Körpers (1) erstreckt,
dadurch gekennzeichnet, daß:
das Siliconröhrchen (9) an jedem seiner Enden mit einem Führungsdraht (6) versehen ist, dessen Durchmesser kleiner als der Innendurchmesser des zylindrischen Hohlkörpers (1) und als die Breite des Loches (2) ist, wobei ein verengter Bereich (7), dessen Durchmesser geringfügig kleiner als die Breite des Schlitzes (5) ist, über einer Länge im wesentlichen gleich der Länge des Schlitzes (5) der Kanüle in dem Führungsdraht (6) benachbart zu seiner Verbindung (8) mit dem Röhrchen (9) vorgesehen ist.
